(19) Europäisches Patentamt
European Patent Office
Office européen des brevets

(11) EP 1 915 994 B1

# (12) EUROPEAN PATENT SPECIFICATION

(45) Date of publication and mention of the grant of the patent:
**01.08.2012 Bulletin 2012/31**

(21) Application number: **06767218.8**

(22) Date of filing: **23.06.2006**

(51) Int Cl.:
*A61K 31/40 (2006.01)* *A61K 31/137 (2006.01)*
*A61K 31/421 (2006.01)* *A61K 31/426 (2006.01)*
*A61K 31/427 (2006.01)* *A61K 31/4439 (2006.01)*
*A61K 31/517 (2006.01)* *A61K 45/00 (2006.01)*
*A61P 1/04 (2006.01)* *A61P 17/06 (2006.01)*
*A61P 19/02 (2006.01)* *A61P 25/00 (2006.01)*
*A61P 37/02 (2006.01)* *A61P 37/06 (2006.01)*
*C07D 207/335 (2006.01)*

(86) International application number:
**PCT/JP2006/312568**

(87) International publication number:
**WO 2006/137509 (28.12.2006 Gazette 2006/52)**

## (54) CYCLIC AMINE DERIVATIVES IN COMBINATION WITH PPAR REGULATORS

CYCLISCHE AMINDERIVATE IN KOMBINATION MIT PPAR-REGULATOREN

DÉRIVÉS D'AMINE CYCLIQUE EN COMBINAISON AVEC DES RÉGULATEURS DU PPAR

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.06.2005 JP 2005185287**

(43) Date of publication of application:
**30.04.2008 Bulletin 2008/18**

(73) Proprietor: **Daiichi Sankyo Company, Limited**
**Chuo-ku**
**Tokyo (JP)**

(72) Inventors:
- **NISHI, Takahide**
**Tokyo 140-8710 (JP)**
- **SHIMOZATO, Takaichi**
**Tokyo 140-8710 (JP)**
- **KAGARI, Takashi**
**Tokyo 140-8710 (JP)**
- **DOI, Hiromi**
**Tokyo 140-8710 (JP)**

(74) Representative: **Wallace, Sheila Jane et al**
**Marks & Clerk LLP**
**90 Long Acre**
**London**
**WC2E 9RA (GB)**

(56) References cited:

| | |
|---|---|
| **EP-A1- 1 772 145** | **EP-A1- 1 782 804** |
| **WO-A1-94/08943** | **WO-A1-96/06068** |
| **WO-A1-97/45141** | **WO-A1-03/059880** |
| **WO-A1-2004/024673** | **WO-A1-2005/105146** |
| **JP-A- 11 080 026** | |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

[TECHNICAL FIELD]

The present invention relates to a pharmaceutical composition comprising a PPAR (peroxisome proliferator-activated receptor) regulator and an amino alcohol derivative having immunosuppressive action or a pharmacologically acceptable salt thereof as active ingredients, which is excellent as a medicament for prophylaxis or treatment of diseases relating to immune action such as rejection in organ transplants or skin grafts, rheumatoid arthritis, psoriasis, inflammatory enteritis, multiple sclerosis and other autoimmune diseases.

[BACKGROUND ART]

Conventionally, in the treatment of rheumatoid arthritis and other autoimmune diseases, anti-inflammatory drugs such as steroids have been used against inflammatory responses, which occur due to immune response disorders. However, problems exist in that they are palliative and in that they may have serious side effects. In addition, it has been reported that immune system disorders are also involved in the onset of diabetes and nephritis (for example, refer to Non-patent Document 1 and Non-patent Document 2); however, no medicament which can improve such disorders has been developed to date.

Immunosuppressive agents such as cyclosporin A (CsA) and tacrolimus (TRL) are extremely important for prevention of rejection in organ transplants and skin grafts, and also for treatment of various autoimmune diseases. However, conventional immunosuppressive agents are known to show toxicity against the kidney and the liver, and thus treatment methods such as by applying steroids in combination have been conducted in order to reduce such side effects. Despite this, alleviation of side effects and a sufficient immunosuppressive effect have not yet necessarily been achieved.

Recently, research and development on the following amino alcohol derivatives and the like as novel immunosuppressive agents have been reported; however, they are not yet actually used in clinical applications.

That is, they are

(1) compounds of the general formula (a) (refer to Patent Document 1):

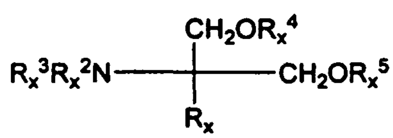

(a)

{wherein $R_x$ is a linear or branched alkyl chain [said carbon chain may have a double bond, a triple bond, an oxygen, a sulfur, -N($R_x^6$)- ($R_x^6$ represents a hydrogen), an allylene which may have a substituent, and a heteroallylene which may have a substituent, and the terminal of said alkyl chain may include an aryl which may have a substituent, a cycloalkyl which may have a substituent, and a heteroaryl which may have a substituent] which may have a substituents, or the like, and $R_x^2$, $R_x^3$, $R_x^4$ and $R_x^5$ may be identical or different, and each represents a hydrogen, an alkyl, or the like};

(2) compounds of the general formula (b) (refer to Patent Document 2):

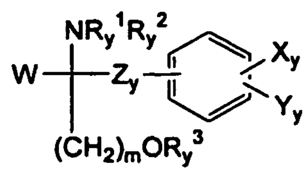

. (b)

[wherein $R_y^1$, $R_y^2$ and $R_y^3$ are hydrogen atoms or the like, W is a hydrogen atom, an alkyl group or the like, $Z_y$ is a single bond or an alkylene group, and $X_y$ is a hydrogen atom or an alkoxy group, $Y_y$ is a hydrogen atom, an alkyl, alkoxy, acyl, acyloxy, amino, or acylamino group or the like];

(3) compounds of the general formula (c) (refer to Patent Document 3):

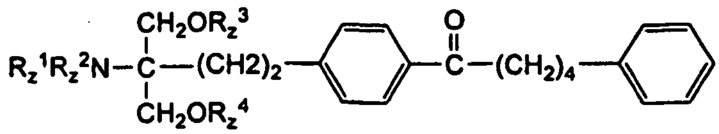

(c)

[wherein $R_z^1$, $R_z^2$, $R_z^3$ and $R_z^4$ are identical or different, and each represents a hydrogen or an acyl group]; and
(4) compounds of the general formula (d) (refer to Patent Document 4 and Patent Document 5):

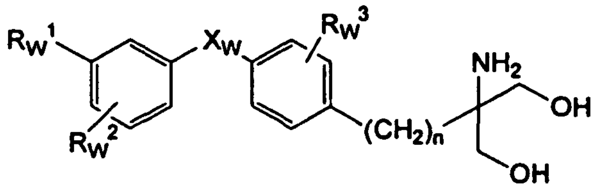

(d)

[wherein $R_w^1$ is a halogen atom, a trihalomethyl group, a hydroxyl group, a lower alkyl group having from 1 to 6 carbon atoms, a phenoxymethyl group or the like; $R_w^2$ is a hydrogen atom, a halogen atom, a trihalomethyl group or the like; $X_w$ is O, S, SO or $SO_2$ ; and n is an integer from 1 to 4].

On the other hand, the present Applicant discloses compounds of the general formula (e) (refer to Patent Document 6) :

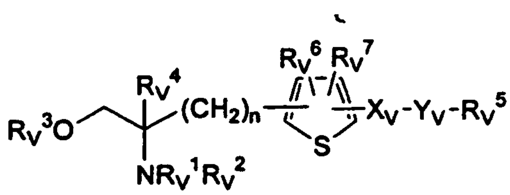

(e)

[wherein $R_v^1$ and $R_v^2$ are a hydrogen atom, an amino-protecting group or the like; $R_v^3$ is a hydrogen atom, a hydroxyl-protecting group and the like; $R_v^4$ is a lower alkyl group; n is an integer from 1 to 6; $X_v$ is an ethylene group or the like; $Y_v$ is a $C_1$-$C_{10}$ alkylene group or the like; and $R_v^5$ is an aryl group, substituted aryl group and the like; $R_v^6$ and $R_v^7$ are hydrogen atoms or the like; here, in the case where $R_v^5$ is a hydrogen atom, $Y_v$ represents a group other than a single bond and a linear $C_1$-$C_{10}$ alkylene group] and
compounds of the following general formula (f) (refer to Patent Document 7):

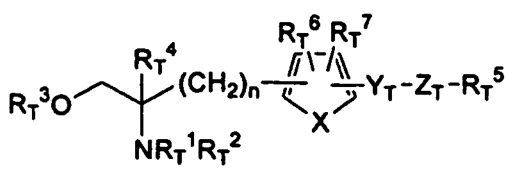

(f)

[wherein $R_T^1$ and $R_T^2$ are a hydrogen atom, an amino-protecting group or the like; $R_T^3$ is a hydrogen atom, a hydroxyl-protecting group or the like; $R_T^4$ is a lower alkyl group; n is an integer from 1 to 6; $X_T$ is an oxygen atom or a nitrogen atom which is unsubstituted or is substituted by a lower alkyl group or the like; $Y_T$ is an ethylene group or the like; Z is an alkylene group having from 1 to 10 carbon atoms or the like; and $R_T^5$ is an aryl group, a substituted aryl group or the like; and $R_T^6$ and $R_T^7$ are hydrogen atoms or the like; here, in the case where $R_T^5$ is a hydrogen atom, $Z_T$ represents a group other than a single bond and a linear $C_1$-$C_{10}$ alkylene group] .

In addition, a report that a PPAR regulator is effective for treatment of autoimmune diseases is known (refer to Patent Document 8).

However, there has not been known a pharmaceutical composition which uses a combination of an aforementioned amino alcohol derivative and a PPAR regulator.

[Non-patent Document 1] Kidney International, vol. 51, 94(1997)
[Non-patent Document 2] Journal of Immunology, vol. 157, 4691(1996)

[Patent Document 1] pamphlet of International Publication WO 94/08943
[Patent Document 2] pamphlet of International Publication WO 96/06068
[Patent Document 3] pamphlet of International Publication WO 98/45249
[Patent Document 4] pamphlet of International Publication WO 03/029184
[Patent Document 5] pamphlet of International Publication WO 03/029205
[Patent Document 6] pamphlet of International Publication WO 02/06228
[Patent Document 7] pamphlet of International Publication WO
[Patent Document 8] pamphlet of International Publication WO 97/45141

[DISCLOSURE OF THE INVENTION]

[Problems to be Solved by the Invention]

An object of the present invention is to provide a pharmaceutical composition which is excellent as a medicament for prophylaxis or treatment of diseases relating to immune action such as rejection in organ transplants or skin grafts, rheumatoid arthritis, psoriasis, inflammatory enteritis, multiple sclerosis and other autoimmune diseases.

[Means for Solving the Problems]

As a result of conducting extensive research with respect to pharmaceutical compositions that have immunosuppressive action, the present inventors found that the pharmaceutical composition according to the present invention possesses low toxicity and excellent immunosuppressive action, exerts enhanced pharmacological effects of both a PPAR regulator and an amino alcohol derivative that are included in the pharmaceutical composition, lowers side effects, and is useful for autoimmune diseases such as systemic lupus erythematosus, rheumatoid arthritis, polymyositis, dermatomyositis, scleroderma, Behcet's disease, Crohn's disease, ulcerative colitis, autoimmune hepatitis, aplastic anemia, exanthema thrombocytopenic purpura, autoimmune hemolytic anemia, multiple sclerosis, autoimmune bullous disease, psoriasis vulgaris, vascular inflammation group, Wegener's granuloma, uveitis, exanthema pneumonitis, Goodpasture's syndrome, sarcoidosis, allergic granulomatous angiitis, bronchial asthma, myocarditis, cardiomyopathy, aortitis syndrome, post-myocardial infarction syndrome, primary pulmonary hypertension, minimal change nephropathy, membranous nephropathy, membranous proliferative nephropathy, focal glomerulosclerosis, crescentic, myasthenia gravis, inflammatory neuropathy, atopic dermatitis, chronic actinic dermatitis, acute polyarthritis, Sydenham's chorea, systemic sclerosis, adult-onset diabetes, insulin-dependent diabetes, juvenile diabetes, atherosclerosis, glomerulonephritis, tubulointerstitial nephritis, primary biliary cirrhosis, primary sclerosing cholangitis, fulminant hepatic failure, viral hepatitis, GVHD, rejection in various organ transplants, contact dermatitis and sepsis, or other diseases related to immunology (especially autoimmune diseases), thereby leading to completion of the present invention.
The present invention provides

(1) a pharmaceutical composition or a kit comprising

one or more PPAR regulators selected from the group consisting of pioglitazone, rosiglitazone and the compound of the formula (II) :

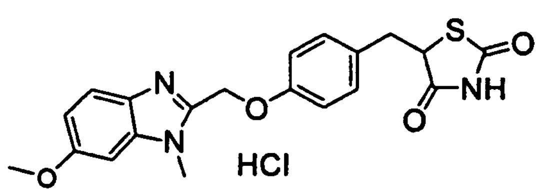

(II)

and
one or more amino alcohol derivatives selected from the group consisting of:
2-amino-2-methyl-4-{1-methyl-5-[5-phenylpentanoyl]pyrrol-2-yl}butan-1-ol,
2-amino-2-methyl-4-{1-methyl-5-[5-(4-methylphenyl)pentanoyl]pyrrol-2-yl}butan-1-ol,
2-amino-2-methyl-4-{1-methyl-5-[5-(3,4-dimethylphenyl)pentanoyl]pyrrol-2-yl}butan-1-ol,
2-amino-2-methyl-4-{1-methyl-5-[5-(3-methyl-4-methoxyphenyl)pentanoyl]pyrrol-2-yl}butan-1-ol,
2-amino-2-methyl-4-{1-methyl-5-[5-(3-methoxy-4-methylphenyl)pentanoyl]pyrrol-2-yl}butan-1-ol,
2-amino-2-methyl-4-{1-methyl-5-[5-(4-cyanophenyl)pentanoyl]pyrrol-2-yl}butan-1-ol,
2-amino-2-methyl-4-{1-methyl-5-[4-(4-methylphenyl)butanoyl]pyrrol-2-yl}butan-1-ol,

2-amino-2-methyl-4-{1-methyl-5-[4-(3,4-dimethylphenyl)butanoyl]pyrrol-2-yl}butan-1-ol,
2-amino-2-methyl-4-{1-methyl-5-[4-(3-methyl-4-methoxyphenyl)butanoyl]pyrrol-2-yl}butan-1-ol,
2-amino-2-methyl-4-{1-methyl-5-[4-(3-methoxy-4-methylphenyl)butanoyl]pyrrol-2-yl}butan-1-ol, or
2-amino-2-methyl-4-{1-methyl-5-[4-(4-cyanophenyl)butanoyl]pyrrol-2-yl}butan-1-ol; and
pharmacologically acceptable salts thereof.

The above-described pharmaceutical composition or kit is preferably

(2) a pharmaceutical composition or kit according to (1), wherein the PPAR regulator is the compound of the formula (II);
(3) a pharmaceutical composition or kit according to (1) or (2), for use in suppression of rejection in organ transplants or skin grafts;
(4) a pharmaceutical composition or kit according to (1) to (3), for use in prophylaxis or treatment of autoimmune disease; and
(5) a pharmaceutical composition or kit according to (4), wherein the autoimmune disease is rheumatoid arthritis, psoriasis, inflammatory enteritis or multiple sclerosis.

In addition, the present invention provides

(6) use of a pharmaceutical composition or kit according to (1) or (2), for the manufacture of a medicament for suppression of rejection in organ transplants or skin grafts;
(7) use of a pharmaceutical composition or kit according to (1) or (2), for the manufacture of a medicament for prophylaxis or treatment of an autoimmune disease; and
(8) use according to (7), wherein the autoimmune disease is rheumatoid arthritis, psoriasis, inflammatory enteritis or multiple sclerosis.

[Effects of the Invention]

The pharmaceutical composition according to the present invention is useful as a pharmaceutical composition which is excellent as a medicament for prophylaxis or treatment of diseases relating to immune action such as rejection in organ transplants or skin grafts, rheumatoid arthritis, psoriasis, inflammatory enteritis, multiple sclerosis and other autoimmune diseases.

[BEST MODE FOR CARRYING OUT THE INVENTION]

"PPAR regulator", which is one of active ingredients of the pharmaceutical composition according to the present invention, includes an antagonist, agonist or the like of a PPAR subtype (for example, $\alpha$ and/or $\gamma$), such as a compound of the formula (II) 5-[4-(6-methoxy-1-methyl-1-H-benzimidazol-2-yl methoxy)benzyl]thiazoline-2,4-dione hydrochloride (pamphlet of International Publication WO 00/71540), ($\pm$)-5-[[4-[2-(5-ethyl-2-pyridinyl)ethoxy]phenyl]methyl]-2,4-thiazolidindione mono hydrochloride (pioglitazone, Japanese Patent Publication No. 1853588; EP-A-193256), and ($\pm$)-5-[4-[2-(methyl-2-pyridinylamino)ethoxy]benzyl]-2,4-thiazolidindione maleic acid (rosiglitazone, pamphlet of International Publication WO 95/21608).
The PPAR regulator is pioglitazone, rosiglitazone or a compound of the formula (II).
Plane structural formulas for the PPAR regulators are given below.

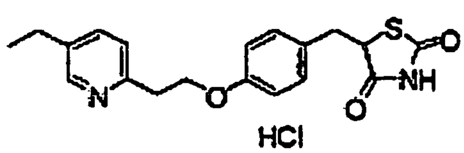

Pioglitazone

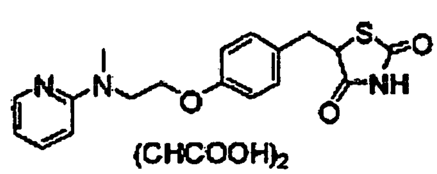

Rosiglitazone

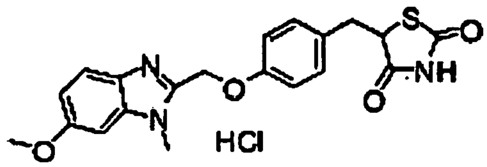

Formula (II)

The compounds as the amino alcohol derivatives, which is one of the active ingredients of the pharmaceutical composition according to the present invention, are

2-amino-2-methyl-4-(-methyl-5-[5-phenylpentanoyl]pyrrol-2-yl)butan-1-ol,
2-amino-2-methyl-4-{1-methyl-5-[5-(4-methylphenyl)pentanoyl]pyrrol-2-yl}butan-1-ol,
2-amino-2-methyl-4-{1-methyl-5-[5-(3,4-dimethylphenyl)pentanoyl]pyrrol-2-yl}butan-1-ol,
2-amino-2-methyl-4-{1-methyl-5-[5-(3-methyl-4-methoxyphenyl)pentanoyl]pyrrol-2-yl}butan-1-ol,
2-amino-2-methyl-4-{1-methyl-5-[5-(3-methoxy-4-methylphenyl)pentanoyl]pyrro.l-2-yl}butan-1-ol,
2-amino-2-methyl-4-{1-methyl-5-[5-(4-cyanophenyl)pentanoyl]pyrrol-2-yl}butan-1-ol,
2-amino-2-methyl-4-{1-methyl-5-[4-(4-methylphenyl)butanoyl]pyrrol-2-yl}butan-1-ol,
2-amino-2-methyl-4-{1-methyl-5-[4-(3,4-dimethylphenyl)butanoyl]pyrrol-2-yl}butan-1-ol,
2-amino-2-methyl-4-{1-methyl-5-[4-(3-methyl-4-methoxyphenyl)butanoyl]pyrrol-2-yl}butan-1-ol,
2-amino-2-methyl-4-{1-methyl-5-[4-(3-methoxy-4-methylphenyl)butanoyl]pyrrol-2-yl}butan-1-ol and
2-amino-2-methyl-4-{1-methyl-5-[4-(4-cyanophenyl)butanoyl]pyrrol-2-yl}butan-1-ol.

Structural formulas for compounds as the amino alcohol derivatives, which is one of the active ingredients of the pharmaceutical composition according to the present invention, are given below.

(Table 1)

| Compound No. | Structural formula |
|---|---|
| 1 | 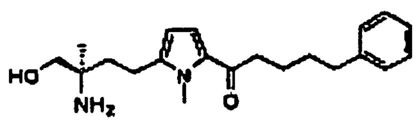 |
| 4 | 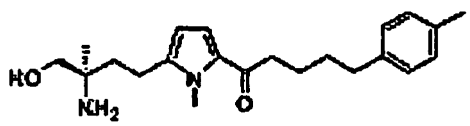 |
| 8 | 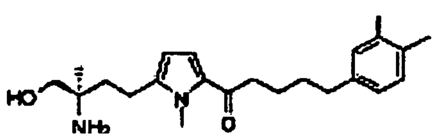 |
| 10 | 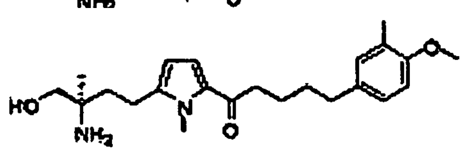 |
| 11 | 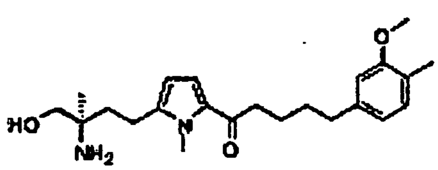 |
| 12 | 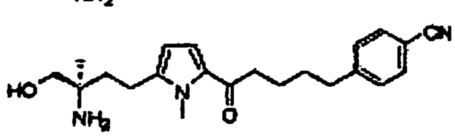 |

(continued)

| Compound No. | Structural formula |
|---|---|
| 15 | 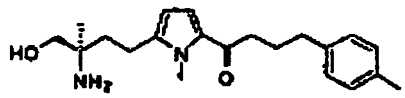 |
| 18 | 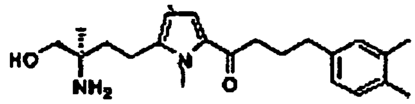 |
| 21 | 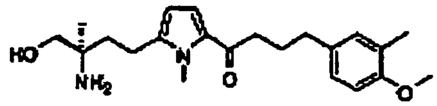 |
| 22 | 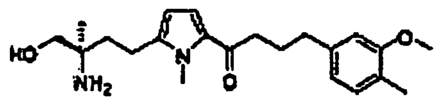 |
| 23 | 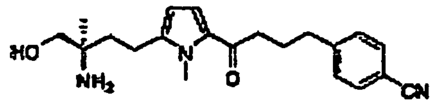 |

The aforementioned "pharmacologically acceptable salts thereof" are salts obtained by reaction with an acid, since the amino alcohol derivatives possess an amino group as a basic group, and include, for example, inorganic acid salts such as hydrohalogenic acid salts, e.g. a hydrofluoric acid salt, a hydrochloric acid salt, a hydrobromic acid salt or a hydroiodic acid salt, a nitric acid salt, a perchloric acid salt, a sulfuric acid salt, a phosphoric acid salt or the like; organic acid salts such as lower alkane sulfonic acid salts, e.g. a methanesulfonic acid salt, a trifluoromethanesulfonic acid salt or an ethanesulfonic acid salt, aryl sulfonic acid salts, e.g. a benzenesulfonic acid salt or a p-toluenesulfonic acid salt, an acetic acid salt, a malic acid salt, a fumaric acid salt, a succinic acid salt, a citric acid salt, an ascorbic acid salt, a tartaric acid salt, an oxalic acid salt or a maleic acid salt; or amino acid salts, e.g. a glycine salt, a lysine salt, an _ arginine salt, an ornithine salt, a glutamic acid salt and an asparatic acid salt, and preferably a hydrochloric acid salt, an acetic acid salt, a fumaric acid salt, a succinic acid salt or a maleic acid salt.

There exists an optical isomer, in cases where there is an asymmetric carbon atom within the molecule of the amino alcohol derivatives, which is one of the active ingredients of the pharmaceutical composition according to the present invention. Among the amino alcohol derivatives, compounds which have an asymmetric carbon atom are represented as a single formula, that is, as the R-configuration. However, depending on the preparation process, there may be cases where a compound with the S-configuration becomes contaminated as a by-product. Therefore, in such cases, the amino alcohol derivatives mainly contain, as optical isomers, the one in the R-configuration but also in part contain the one in the S-configuration.

When the amino alcohol derivatives and pharmacologically acceptable salts thereof are exposed to the atmosphere or are recrystallized, they may absorb moisture, resulting in cases such as addition of adsorbed water and generation of hydrates. Such hydrates are also embraced in the pharmacologically acceptable salts of the amino alcohol derivatives used in the present invention.

The pharmaceutical composition according to the present invention exerts enhanced pharmacological effects of both the PPAR regulator and the amino alcohol derivatives that are contained in the composition, shows excellent immunosuppressive action, as well as lowering side effects and having low toxicity, thereby being useful as a prophylactic drug or a therapeutic drug (especially a therapeutic drug) for autoimmune diseases such as systemic lupus erythematosus, rheumatoid arthritis, polymyositis, dermatomyositis, scleroderma, Behcet's disease, Crohn's disease, ulcerative colitis, autoimmune hepatitis, aplastic anemia, exanthema thrombocytopenic purpura, autoimmune hemolytic anemia, multiple sclerosis, autoimmune bullous disease, psoriasis vulgaris, vascular inflammation group, Wegener's granuloma, uveitis, exanthema pneumonitis, Goodpasture's syndrome, sarcoidosis, allergic granulomatous angiitis, bronchial asthma, myocarditis, cardiomyopathy, aortitis syndrome, post-myocardial infarction syndrome, primary pulmonary hypertension, minimal change nephropathy, membranous nephropathy, membranous proliferative nephropathy, focal glomerulosclerosis, crescentic, myasthenia gravis, inflammatory neuropathy, atopic dermatitis, chronic actinic dermatitis, acute polyarthritis, Sydenham's chorea, systemic sclerosis, adult-onset diabetes, insulin-dependent diabetes, juvenile diabetes, atherosclerosis, glomerulonephritis, tubulointerstitial nephritis, primary biliary cirrhosis, primary sclerosing cholangitis, fulminant hepatic failure, viral hepatitis, GVHD, rejection in various kinds of organ transplants, contact dermatitis and sepsis, or other diseases related to immunology (especially autoimmune diseases).

In the case where the pharmaceutical composition according to the present invention is used as a prophylactic

drug or a therapeutic drug for the aforementioned diseases, the pharmaceutical composition according to the present invention can be mixed with a pharmacologically acceptable excipient, diluent or the like, and can be administered as an oral drug by a tablet, a capsule, granules, powders or syrup, or as a parenteral drug by injection or suppository.

These pharmaceutical preparations are prepared in accordance with known processes by using additives, including excipients (for example, organic excipients such as sugar derivatives, e.g. lactose, sucrose, glucose, mannitol or sorbitol; starch derivatives, e.g. corn starch, potato starch, α-starch or dextrin; cellulose derivatives, e.g. crystalline cellulose; gum arabic; dextrane; or pullulan, and inorganic excipients such as silicate derivatives, e.g. light silicic anhydride, synthetic aluminum silicate, calcium silicate, meta magnesium aluminate; phosphates, e.g. calcium hydrogenphosphate; carbonates, e.g. calcium carbonate; salts of sulfuric acid such as calcium sulfate, can be mentioned), lubricants (for example, stearic acid, stearic acid metal salts such as calcium stearate or magnesium stearate; talc; colloid silica; waxes such as bee gum or spermaceti, boric acid; adipic acid; sulfates such as sodium sulfate; glycol; fumaric acid; sodium benzoate; DL leucine; sodium salt of fatty acid; lauryl sulfates such as sodium lauryl sulfate or magnesium lauryl sulfate; silicic acids such as silicic anhydride or silicate hydrate; and the aforementioned starch derivatives can be mentioned), binders (for example, hydroxypropyl cellulose, hydroxypropyl methyl cellulose, polyvinylpyrrolidone, macrogol and compounds similar to the aforementioned excipients can be mentioned), disintegrants (for example, cellulose derivatives such as low-substituted hydroxypropyl cellulose, carboxymethyl cellulose, calcium carboxymethyl cellulose or internally crosslinked sodium carboxymethyl cellulose; or chemically modified starches or celluloses such as carboxymethyl starch, sodium carboxymethyl starch or crosslinked polyvinylpyrrolidone can be mentioned), stabilizers (for example, paraoxybenzoic acid esters such as methyl paraben or propyl paraben; alcohols such as chlorobutanol, benzyl alcohol or phenyl ethyl alcohol, benzalkonium chloride; phenols such as phenol or cresol; thimerosal; dehydroacetic acid; and sorbic acid can be mentioned) and corrigents (for example, commonly used sweeteners, acidifiers or fragrances can be mentioned) or diluents.

With respect to the pharmaceutical composition according to the present invention, 1 or 2 or more PPAR regulators selected from the aforementioned specific group and 1 or 2 or more selected from the group consisting of the aforementioned amino alcohol derivatives and pharmacologically acceptable salts thereof can be administered either simultaneously or separately at a time interval; however, from the clinical perspective, it is convenient to administer them simultaneously, and thus it is preferable that the PPAR regulator and the amino alcohol derivative or pharmacologically acceptable salt thereof be administered as a formulation.

Concerning the pharmaceutical technology, in cases where it is not preferable for both of the compounds to be physically mixed together, each of the monotherapies can be administered simultaneously or at a time interval.

In the present invention, to "administer separately at a time interval" is not specifically limited so long as it is in an administration form which is capable of being administered at different times. For example, 1 or 2 or more selected from the group consisting of the amino alcohol derivatives and pharmacologically acceptable salts thereof may be administered first, and then after a predetermined time, 1 or 2 or more PPAR regulators selected from the specific group may be administered; or alternatively, the PPAR regulators may be administered first, and then after a predetermined time, the aforementioned amino alcohol derivatives or pharmacologically acceptable salts thereof may be administered.

In the present invention, "treatment" means to cure or improve a disease or symptoms, or to alleviate symptoms, and "prophylaxis" means to prevent an expression of a disease or symptoms.

In the present invention, "formulation" refers to a single composition in which a plurality of ingredients are mixed.

In the present invention, "kit" refers to the case in which a plurality of individual compositions are used as one set.

In the present invention, the maximum administration interval of the aforementioned two types of drugs, which can achieve the excellent effect provided by the PPAR regulator and at least one compound selected from the group consisting of the aforementioned amino alcohol derivatives and pharmacologically acceptable salts thereof, can be confirmed by clinical or animal experiments.

The administration amount and administration ratio of the active ingredients of the pharmaceutical composition according to the present invention, which are the 1 or 2 or more PPAR regulators selected from the aforementioned specific group and 1 or 2 or more compounds selected from the group consisting of the aforementioned amino alcohol derivatives and pharmacologically acceptable salts thereof, may vary depending on various conditions such as the activity of each of the drugs, symptoms, age and weight of the patient, and the like.

Although the single dosage of the PPAR regulator as an active ingredient varies depending on symptoms, age and the like,

in the case of oral administration, it is 0.001 mg/kg to 1.7 mg/kg, preferably 0.002 mg/kg to 0.83 mg/kg for an adult human, and

in the case of intravenous administration, it is 0.0005 mg/kg to 0.8 mg/kg, preferably 0.001 mg/kg to 0.4 mg/kg for an adult human.

Although the single dosage of the aforementioned amino alcohol derivatives and pharmacologically acceptable salts thereof as an active ingredient varies depending on symptoms, age and the like, it is for example, 0.0001 mg/kg to 1.0 mg/kg, preferably 0.001 mg/kg to 0.1 mg/kg for an adult human, irrespective of the route of administration such

as oral administration or intravenous administration.

With respect to oral administration, the number of administrations is generally 1 to 3 times a day, or the number of administration may be decreased depending on circumstances, such as once a week. The number of administrations is preferably once a day to once a week, and more preferably once a day to once every three days.

In addition, the administration amount ratio of the 1 or 2 or more PPAR regulators selected from the aforementioned specific group and the 1 or 2 or more compounds selected from the group consisting of the aforementioned amino alcohol derivatives and pharmacologically acceptable salts thereof may also vary to a large extent; for example, the administration amount ratio of the aforementioned PPAR regulator and the compound selected from the group consisting of the 1 or 2 or more selected from the group consisting of the amino alcohol derivatives and pharmacologically acceptable salts thereof may be in the range of 1:2 to 500:1 by weight.

Test examples and preparation examples will be given hereinafter, and the present invention will be described in more detail; however, the scope of the present invention should not be limited thereto.

(Test Example)

(Test Example 1) Evaluation of Anti-arthritic Action

A rat adjuvant arthritis model developing arthritis that is similar to human rheumatoid arthritis was used, and effect of the pharmaceutical composition according to the present invention with respect to the arthritis was evaluated by using footpad volume increase suppression rate as an indicator. Eight-week old female Lewis rats were used in the experiment.

(1) Preparation of Adjuvant

Heat killed Mycobacterium butyricum was ground in an agate mortar, then suspended in dry heat sterilized liquid paraffin so as to have a concentration of 2 mg/ml, and treated with sonication to prepare an adjuvant.

(2) Preparation of Test Compound

The test compound was suspended or dissolved in a 0.5% tragacanth solution. As the test compound, the compound of Compound No. 1 described in Table 1 was used as the amino alcohol derivative, and the compound of the formula (II) was used as the PPAR regulator.

(3) Induction of Adjuvant Arthritis

0.05 ml of the adjuvant prepared in (1) was injected subcutaneously into the footpad of right hind leg of rats, with respect to a control group and a test compound administration group. Here, 5 rats were used for each group. In addition, a normal control group was provided as a group without injection of the adjuvant. (4) Administration of Test Compound

The test compound prepared in (2) was orally administrated once a day in the amount of 5 ml/kg, after 18 days from the day of adjuvant injection. The control group was administered with only the 0.5% tragacanth solution in a similar manner.

(5) Calculation of Footpad Volume Increase Suppression Ratio of Test Compound

After the start of administration, on day 11 and day 18, the volume of the right hind leg was measured by a leg volume measuring device, and the mean value of the swelling volume was calculated for each group.

Results of the experiment are given in Table 2. The footpad volume increase suppression ratio (%) was calculated by the following equation, and evaluated.

$$\text{Footpad volume increase suppression ratio (\%)} = \left(1 - \frac{[\text{footpad volume of test compound administration group}] - [\text{footpad volume of normal control group}]}{[\text{footpad volume of control group}] - [\text{footpad volume of normal control group}]}\right) \times 100$$

(Table 2)

| Test Compound (administration amount mg/kg) | Footpad volume increase suppression ratio (%) | |
|---|---|---|
| | After 11 days | After 18 days |
| Compound No. 1 (0.1) | 43.0 | 44.8 |
| Formula (II) (10.0) | 24.7 | 30.1 |
| Compound No. 1 (0.1) + Formula (II) (10.0) | 53.2 | 49.4 |

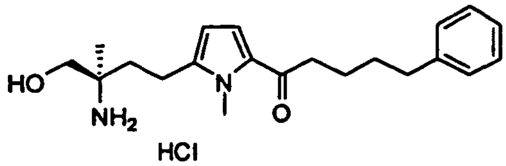

Compound No. 1 (Table 1)

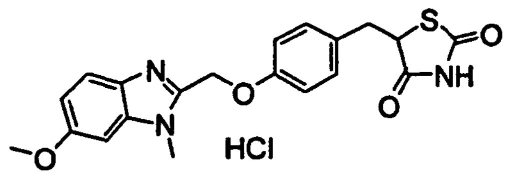

Formula (II)

The results of the experiment showed that when compared with the case where the Compound No. 1 or the compound of the formula (II) are each administered separately, the footpad volume increase suppression ratio is higher when both of the compounds are used in combination, and that the pharmaceutical composition according to the present invention, which contains the amino alcohol derivative and the PPAR regulator, has excellent anti-arthritis action.

(Test Example 2) Evaluation of Suppression Activity with Respect to Rat HvGR (Host versus Graft Reaction)

Two families of rat [Lewis (male, 6 weeks old, Charles River Laboratories Japan, Inc.) and WKAH/Hkm (male, 7 weeks old, Japan SLC, Inc.)] are used. Five rats (hosts) are used for each group.

(1) Induction of HvGR

Spleen cells are isolated from the spleens of the WKAH/Hkm rats and the Lewis rats, and are suspended in RPMI1640 medium (Life Technologies, Inc.) so as to have a concentration of 1 x $10^8$ cells/ml. 0.1 ml of spleen cell suspension (1 **x** $10^7$ cells as the number of spleen cells) of WKAH/Hkm rats or Lewis rats are injected subcutaneously into the footpad of both hind legs of Lewis rats. (2) Administration of Test Compound

The test compounds are used as a suspension in a 0.5% tragacanth solution. The suspension of the test compound (5 ml per 1 kg of rat's weight) is orally administered to the test compound administration group (Lewis rat that is injected with spleen cells of WKAH/Hkm rat and is administered with the test compound), once a day, for four days successively from the day the spleen cells are injected. Here, the syngeneic group (Lewis rat that is injected with spleen cells of Lewis rat) and the control group (Lewis rat that is injected with the spleen cells of WKAH/Hkm rat and is not administered with the test compound) are orally administered with 0.5% tragacanth solution in place of the test compound.

(3) Measurement of Suppression Activity with Respect to HvGR

The mean popliteal lymph node weight is subtracted from the popliteal lymph node weight, with respect to each individual for each of the administration groups ("popliteal lymph weight by HvGR"), and the suppression ratio is calculated from "popliteal lymph weight by HvGR" of each individual of the compound administration group with respect to the mean "popliteal lymph weight by HvGR" of the control group.

(Test Example 3) Evaluation of Peripheral Blood Lymphocyte Decreasing Action in Rat

LEW rats (male, 5 weeks old, Charles River Laboratories Japan, Inc.) are used. 5 rats per group are used.

(1) Administration of Test Compound

The test compound is suspended in a 1% tragacanth solution (solvent). The test compound suspension is

orally administered forcedly at the rate of 5 ml per 1 kg of the rat's weight.

Here, solvent is administered to the normal group, in place of the test compound suspension.

(2) Measurement of Peripheral Blood Lymphocytes

After 3 hours from administration of the solvent or the test compound suspension, blood is collected from the inferior vena cava under etherization, and is transferred to a tube containing EDTA. The absolute lymphocytic count of the collected blood is measured by a hematology examination device. The peripheral blood lymphocyte decreasing action is calculated as the relative value (%), taking the lymphocytic count of the normal group as 100%.

(Preparation Example)

Ingredients contained in 1 tablet (150 mg)

| | |
|---|---|
| PPAR regulator | 50 mg |
| Immunosuppressive agent | 10 mg |
| Lactose | 113 mg |
| Corn starch | 25 mg |
| Magnesium stearate | 2 mg |
| | 200 mg |

The aforementioned powders are mixed, and tablets of 200 mg per tablet are prepared in accordance with the "tablets" section in General Rules for Preparation of Japanese Pharmacopoeia, by tableting with a tableting machine.

## Claims

**1.** A pharmaceutical composition comprising
one or more PPAR regulators selected from the group consisting of pioglitazone, rosiglitazone and the compound of the formula (II):

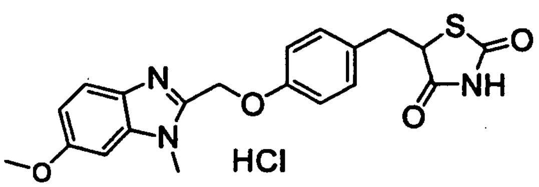

(II)

and
one or more amino alcohol derivatives selected from the group consisting of:

2-amino-2-methyl-4-{1-methyl-5-[5-phenylpentanoyl]pyrrol-2-yl}butan-1-ol,
2-amino-2-methyl-4-{1-methyl-5-[5-(4-methylphenyl)pentanoyl]pyrrol-2-yl}butan-1-ol,
2-amino-2-methyl-4-{1-methyl-5-[5-(3,4-dimethylphenyl)pentanoyl]pyrrol-2-yl}butan-1-ol,
2-amino-2-methyl-4-{1-methyl-5-[5-(3-methyl-4-methoxyphenyl)pentanoyl]pyrrol-2-yl}butan-1-ol,
2-amino-2-methyl-4-{l-methyl-5-[5-(3-methoxy-4-methylphenyl)pentanoyl]pyrrol-2-yl}butan-1-ol,
2-amino-2-methyl-4-{1-methyl-5-[5-(4-cyanophenyl)pentanoyl]pyrrol-2-yl}butan-1-ol,
2-amino-2-methyl-4-{1-methyl-5-[4-(4-methylphenyl)butanoyl]pyrrol-2-yl}butan-1-ol,
2-amino-2-methyl-4-{1-methyl-5-[4-(3,4-dimethylphenyl)butanoyl]pyrrol-2-yl}butan-1-ol,
2-amino-2-methyl-4-{1-methyl-5-[4-(3-methyl-4-methoxyphenyl)butanoyl]pyrrol-2-yl}butan-1-ol,
2-amino-2-methyl-4-{1-methyl-5-[4-(3-methoxy-4-methylphenyl)butanoyl]pyrrol-2-yl}butan-1-ol, or
2-amino-2-methyl-4-{1-methyl-5-[4-(4-cyanophenyl)butanoy]pyrrol-2-yl}butan-1-ol; and
pharmacologically acceptable salts thereof.

**2.** A pharmaceutical composition according to claim 1, wherein the PPAR regulator is the compound of the formula (II).

**3.** A kit comprising the PPAR regulator(s) and the amino alcohol derivative(s) as defined in Claim 1 or Claim 2.

**4.** A pharmaceutical composition according to claim 1 or claim 2, or a kit according to claim 3, for use in suppression of rejection in organ transplants or skin grafts.

**5.** A pharmaceutical composition according to claim 1 or claim 2, or a kit according to claim 3, for use in prophylaxis or treatment of autoimmune disease.

**6.** A pharmaceutical composition or kit according to claim 5, wherein the autoimmune disease is rheumatoid arthritis, psoriasis, inflammatory enteritis or multiple sclerosis.

**7.** Use of a pharmaceutical composition according to claim 1 or claim 2, or of a kit according to claim 3, for the manufacture of a medicament for suppression of rejection in organ transplants or skin grafts.

**8.** Use of a pharmaceutical composition according to claim 1 or claim 2, or of a kit according to claim 3, for the manufacture of a medicament for prophylaxis or treatment of an autoimmune disease.

**9.** Use according to claim 8, wherein the autoimmune disease is rheumatoid arthritis, psoriasis, inflammatory enteritis or multiple sclerosis.

## Patentansprüche

**1.** Pharmazeutische Zusammensetzung, umfassend
einen oder mehrere PPAR-Regulatoren, ausgewählt aus der Gruppe bestehend aus Pioglitazon, Rosiglitazon und der Verbindung der Formel (II):

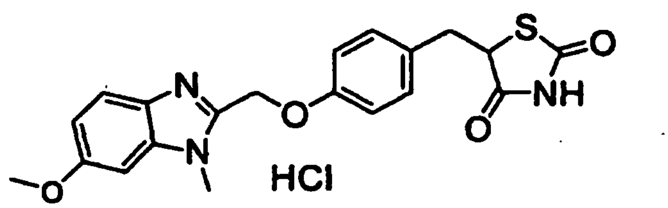

**(II)**

und
ein oder mehrere Aminoalkoholderivate, ausgewählt aus der Gruppe bestehend aus:
2-Amino-2-methyl-4-{1-methyl-5-[5-phenylpentanoyl]pyrrol-2-yl}butan-1-ol, 2-Amino-2-methyl-4-{1-methyl-5-[5-(4-methylphenyl)-pentanoyl]pyrrol-2-yl}butan-1-ol,
2-Amino-2-methyl-4-(1-methyl-5-[5-(3,4-dimethylphenyl)pentanoyl]pyrrol-2-yl}-butan-1-ol,
2-Amino-2-methyl-4-{1-methyl-5-[5-(3-methyl-4-methoxyphenyl)pentanoyl]pyrrol-2-yl}butan-1-ol,
2-Amino-2-methyl-4-{1-methyl-5-[5-(3-methoxy-4-methylphenyl)pentanoyl]pyrrol-2-yl}butan-1-ol,
2-Amino-2-methyl-4-{1-methyl-5-[5-(4-cyanophenyl)pentanoyl]pyrrol-2-yl}butan-1-ol,
2-Amino-2-methyl-4-{1-methyl-5-[4-(4-methylphenyl)butanoyl]pyrrol-2-yl}butan-1-ol,
2-Amino-2-methyl-4-{1-methyl-5-[4-(3,4-dimethylphenyl)butanoyl]pyrrol-2-yl}-butan-1-ol,
2-Amino-2-methyl-4-{1-methyl-5-[4-(3-methyl-4-methoxyphenyl)butanoyl]pyrrol-2-yl}butan-1-ol,
2-Amino-2-methyl-4-{1-methyl-5-[4-(3-methoxy-4-methylphenyl)butanoyl]pyrrol-2-yl}butan-1-ol, oder
2-Amino-2-methyl-4-{1-methyl-5-[4-(4-cyanophenyl)butanoyl]pyrrol-2-yl}butan-1-ol; und
pharmakologisch akzeptable Salze davon.

**2.** Pharmazeutische Zusammensetzung nach Anspruch 1, wobei der PPAR-Regulator die Verbindung der Formel (II) ist.

**3.** Kit, umfassend den PPAR-Regulator bzw. die PPAR-Regulatoren und das Aminosäurederivat bzw. die Aminosäurederivate nach Anspruch 1 oder 2.

4. Pharmazeutische Zusammensetzung nach Anspruch 1 oder 2 oder ein Kit nach Anspruch 3 zur Verwendung bei der Suppression des Abstoßens bei Organ- oder Hauttransplantaten.

5. Pharmazeutische Zusammensetzung nach Anspruch 1 oder 2 oder ein Kit nach Anspruch 3 zur Verwendung bei der Prophylaxe oder Behandlung einer Autoimmunerkrankung.

6. Pharmazeutische Zusammensetzung oder Kit nach Anspruch 5, wobei die Autoimmunerkrankung rheumatoide Arthritis, Psoriasis, entzündliche Enteritis oder Multiple Sklerose ist.

7. Verwendung einer pharmazeutischen Zusammensetzung nach Anspruch 1 oder 2 oder eines Kits nach Anspruch 3 zur Herstellung eines Medikaments für die Suppression des Abstoßens bei Organ- oder Hauttransplantaten.

8. Verwendung einer pharmazeutischen Zusammensetzung nach Anspruch 1 oder 2 oder eines Kits nach Anspruch 3 zur Herstellung eines Medikaments für die Prophylaxe oder Behandlung einer Autoimmunerkrankung.

9. Verwendung nach Anspruch 8, wobei die Autoimmunerkrankung rheumatoide Arthritis, Psoriasis, entzündliche Enteritis oder Multiple Sklerose ist.

## Revendications

1. Composition pharmaceutique comprenant :

un ou plusieurs régulateurs de PPAR choisis dans le groupe constitué par la pioglitazone, la rosiglitazone et le composé de formule (II) :

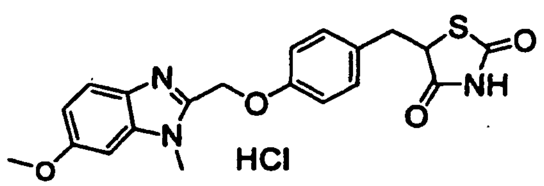

(II)

et
un ou plusieurs dérivés d'alcools aminés choisis dans le groupe constitué par :
le 2-amino-2-méthyl-4-{1-méthyl-5-[5-phénylpentanoyl]pyrrol-2-yl}butan-1-ol,
le 2-amino-2-méthyl-4-{1-méthyl-5-[5-(4-méthylphényl)pentanoyl]pyrrol-2-yl}butan-1-ol,
le 2-amino-2-méthyl-4-{1-méthyl-5-[5-(3,4-diméthylphényl)pentanoyl]pyrrol-2-yl}butan-1-ol,
le 2-amino-2-méthyl-4-{1-méthyl-5-[5-(3-méthyl-4-méthoxyphényl)pentanoyl]-pyrrol-2-yl}butan-1-ol,
le 2-amino-2-méthyl-4-{1-méthyl-5-[5-(3-méthoxy-4-méthylphényl)pentanoyl]-pyrrol-2-yl}butan-1-ol,
le 2-amino-2-méthyl-4-{1-méthyl-5-[5-(4-cyanophényl)pentanoyl]pyrrol-2-yl}butan-1-ol,
le 2-amino-2-méthyl-4-{1-méthyl-5-[4-(4-méthylphényl)butanoyl]pyrrol-2-yl}butan-1-ol,
le 2-amino-2-méthyl-4-{1-méthyl-5-[4-(3,4-diméthylphényl)butanoyl]pyrrol-2-yl}bu-tan-1-ol,
le 2-amino-2-méthyl-4-{1-méthyl-5-[4-(3-méthyl-4-méthoxyphényl)butanoyl]pyrrol-2-yl}butan-1-ol,
le 2-amino-2-méthyl-4-{1-méthyl-5-[4-(3-méthoxy-4-méthylphényl)butanoyl]pyrrol-2-yl}butan-1-ol ou
le 2-amino-2-méthyl-4-{1-méthyl-5-[4-(4-cyanophényl)butanoyl]pyrrol-2-yl}butan-1-ol ; et
leurs sels pharmacologiquement acceptables.

2. Composition pharmaceutique selon la revendication 1, dans laquelle le régulateur de PPAR est le composé de formule (II).

3. Kit comprenant le ou les régulateurs de PPAR et le ou les dérivés d'alcools aminés tels que définis dans la revendication 1 ou la revendication 2.

4. Composition pharmaceutique selon la revendication 1 ou la revendication 2, ou kit selon la revendication 3, pour une utilisation pour la suppression du rejet lors de transplantations d'organes ou de greffes de peau.

5. Composition pharmaceutique selon la revendication 1 ou la revendication 2, ou kit selon la revendication 3, pour

une utilisation pour la prophylaxie ou le traitement d'une maladie auto-immune.

6. Composition pharmaceutique ou kit selon la revendication 5, dans laquelle ou dans lequel la maladie auto-immune est la polyarthrite rhumatoïde, le psoriasis, l'entérite inflammatoire ou la sclérose en plaques.

7. Utilisation d'une composition pharmaceutique selon la revendication 1 ou la revendication 2, ou d'un kit selon la revendication 3, pour la fabrication d'un médicament pour la suppression du rejet lors de transplantations d'organes ou de greffes de peau.

8. Utilisation d'une composition pharmaceutique selon la revendication 1 ou la revendication 2, ou d'un kit selon la revendication 3, pour la fabrication d'un médicament pour la prophylaxie ou le traitement d'une maladie auto-immune.

9. Utilisation selon la revendication 8, dans laquelle la maladie auto-immune est la polyarthrite rhumatoïde, le psoriasis, l'entérite inflammatoire ou la sclérose en plaques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9408943 A
- WO 9606068 A
- WO 9845249 A
- WO 03029184 A
- WO 03029205 A
- WO 0206228 A
- WO 9745141 A
- WO 0071540 A
- JP 1853588 A
- EP 193256 A
- WO 9521608 A


### Non-patent literature cited in the description


- *Kidney International,* 1997, vol. 51, 94
- *Journal of Immunology,* 1996, vol. 157, 4691